(19) Europäisches Patentamt
European Patent Office
Office européen des brevets

(11) Veröffentlichungsnummer: **0 311 086 A1**

(12) **EUROPÄISCHE PATENTANMELDUNG**

(21) Anmeldenummer: 88116596.3

(22) Anmeldetag: 06.10.88

(51) Int. Cl.4: **C07D 275/06 , C07F 7/10 , C07B 39/00 , C07D 513/04 , C07D 279/02 , C07D 281/02**

(30) Priorität: 09.10.87 CH 3956/87

(43) Veröffentlichungstag der Anmeldung: 12.04.89 Patentblatt 89/15

(84) Benannte Vertragsstaaten: BE CH DE FR GB IT LI NL

(71) Anmelder: **CIBA-GEIGY AG**
Klybeckstrasse 141
CH-4002 Basel(CH)

(72) Erfinder: **Allmendinger, Thomas, Dr.**
Schloss-Strasse 43
D-7853 Steinen(DE)
Erfinder: **Differding, Edmond, Dr.**
Rottmannsbodenstrasse 4
CH-4102 Binningen(CH)
Erfinder: **Lang, Robert Werner, Dr.**
Hagenbachweg 10
CH-4133 Pratteln(CH)

(74) Vertreter: **Zumstein, Fritz, Dr. et al**
Bräuhausstrasse 4
D-8000 München 2(DE)

(54) **N-fluorierte Sulfonamide, Verfahren zu deren Herstellung und deren Verwendung.**

(57) Verbindungen der Formel I

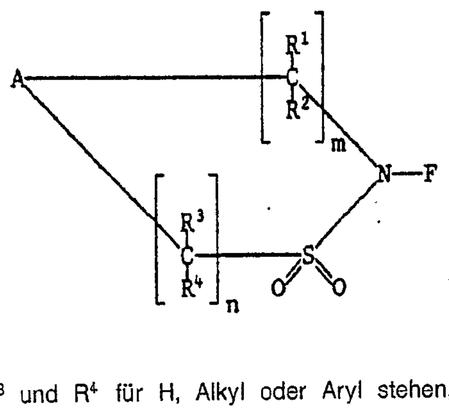

(I),

worin $R^1$, $R^2$, $R^3$ und $R^4$ für H, Alkyl oder Aryl stehen, m und n 0 oder 1 bedeuten, und A einen zweiwertigen organischen Rest darstellt, der mit der $-(CR^1R^2)_m NF-SO_2-(CR^3R^4)_n$ Gruppen einen 5- bis 8-gliedrigen Ring bildet, eignen sich hervorragend als C-Fluorierungsmittel, insbesondere als stereoselektive Fluorierungsmittel, wenn die Verbindungen ein chirales C-Atom enthalten, und in einer optisch aktiven Form vorliegen.

EP 0 311 086 A1

Xerox Copy Centre

## N-fluorierte Sulfonamide, Verfahren zu deren Herstellung und deren Verwendung

Die Erfindung betrifft cyclische N-fluorierte Sulfonamide, cyclische N-silylierte Sulfonamide als Zwischenprodukte, ein Verfahren zu deren Herstellung durch direkte Fluorierung, und deren Verwendung zur C-Fluorierung.

In der US-A-4 479 901 sind N-substituierte N-Fluor-arylsulfonamide beschrieben, die zur Fluorierung von Carbanionen verwendet werden können. Die Direktfluorierung von entsprechenden N-H-Sulfonamiden führt auch mit stark verdünnten $F_2/N_2$-Gasgemischen und bei relativ niedrigen Reaktionstemperaturen ohne HF-Fänger infolge HF-Bildung zu Nebenreaktionen, welche sich in allgemein sehr geringen Ausbeuten widerspiegeln. Im J. Am. Chem. Soc., Vol. 108, Nr. 9, S. 2445-2447, (1986) wird die stereospezifische Synthese von Alkenylfluoriden durch die Umsetzung von Lithiumalkenyl mit N-t-Butyl-N-fluor-phenylsulfonamid beschrieben.

In der EP-A-0 211 578 werden perfluorierte N-fluorierte Bis-sulfonamide beschrieben, die zur elektrophilen C-Fluorierung von Aromaten und zur Fluorierung von Carbanionen verwendet werden können. Die Verbindungen sind relativ instabil und schon bei deren Herstellung muss bei sehr tiefen Temperaturen und mit besonderen Vorsichtsmassnahmen gearbeitet werden.

Ein Gegenstand der Erfindung sind Verbindungen der Formel I

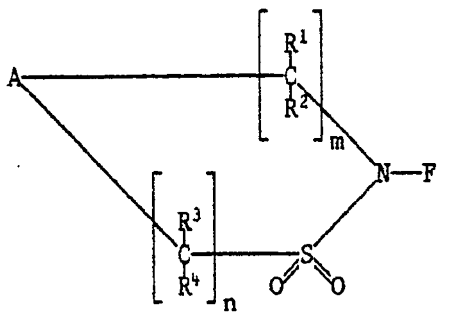

(I),

worin

$R^1$, $R^2$, $R^3$ und $R^4$ unabhängig voneinander für H, lineares oder verzweigtes $C_1$-$C_{18}$-Alkyl oder $C_6$-$C_{10}$-Aryl stehen, die unsubstituiert sind oder das Alkyl mit Halogen, $C_6$-$C_{10}$-Aryl oder -Aryloxy, $C_1$-$C_6$-Alkoxy oder Sekundäramino und das Aryl mit $C_1$-$C_{12}$-Alkyl, -Halogenalkyl oder -Alkoxy, Halogen, Phenyl, Phenoxy oder Sekundäramino substituiert ist. m und n unabhängig voneinander für 0 oder 1 stehen, und A einen zweiwertigen organischen Rest bedeutet, der mit der $\{CR^1R^2\}_m$ NF-$SO_2$$\{CR^3R^4\}_n$ Gruppe einen 5- bis 8-gliedrigen Ring bildet und der Rest A ausgewählt ist aus der Gruppe

a) $C_1$-$C_6$-Alkylen, $C_2$-$C_6$-Alkenylen, $C_4$-$C_6$-Alkdienylen, $C_6$-Alktrienylen, $C_5$-$C_8$-Cycloalkylen, $C_5$-$C_8$-Cycloalkenylen, $C_5$-$C_8$-Cycloalkdienylen oder $C_7$-$C_8$-Cycloalktrienylen, die mit einem carbo- oder heterocyclischen Ring kondensiert oder die cycloaliphatischen Reste mit einer $\{CR^5R^6\}_x$$^5$ und $R^6$ unabhängig H oder $C_1$-$C_4$-Alkyl darstellen und x für 1 oder 2 steht, \

b) $C_6$-$C_{14}$-Arylen oder Heteroarylen mit 5 oder 6 Ringatomen und 1 oder 2 der Heteroatome O, S und tertiäres N, wobei der Rest A unsubstituiert oder ein- oder mehrfach mit $C_1$-$C_6$-Alkyl oder -Alkoxy, Sekundäramino, Phenyl, Phenoxy, Cl, Br und der Rest A als cyclischer Rest auch mit F substituiert ist.

$R^1$, $R^2$, $R^3$ und $R^4$ können lineares oder verzweigtes Alkyl mit vorzugsweise 1 bis 12, besonders 1 bis 6, und insbesondere 1 bis 4 C-Atomen sein. Beispiele sind Methyl, Ethyl und die Isomeren von Propyl, Butyl, Pentyl, Hexyl, Heptyl, Octyl, Nonyl, Decyl, Undecyl, Dodecyl, Tridecyl, Tetradecyl, Pentadecyl, Hexadecyl, Heptadecyl und Octadecyl. Bevorzugt sind Hexyl, Pentyl, n-, i- und t-Butyl, n- und i-Propyl, Ethyl und Methyl. Besonders bevorzugt sind Methyl und Ethyl.

Bei $R^1$, $R^2$, $R^3$ und $R^4$ kann es sich um $C_6$-$C_{10}$-Aryl handeln. Bevorzugte Beispiele sind Naphthyl und insbesondere Phenyl.

$R^1$, $R^2$, $R^3$ und $R^4$ können in der Bedeutung von Alkyl substituiert sein, mit Halogen, vorzugsweise Br, Cl und F; $C_6$-$C_{10}$-Aryl oder -Aryloxy, z.B. Phenyl oder Phenyloxy; $C_1$-$C_6$-Alkoxy, z.B. Methoxy, Ethoxy, n- oder i-Propoxy, n-, i- oder t-Butoxy; Sekundäramino, z.B. $(C_1$-$C_6$-$Alkyl)_2N$-, worin das Alkyl bevorzugt Methyl, Ethyl, Propyl oder Butyl ist; und in der Bedeutung von Aryl mit $C_1$-$C_{12}$-, bevorzugt $C_1$-$C_6$ und

besonders $C_1$-$C_4$-Alkyl, z.B. Butyl, Propyl und insbesondere Ethyl und Methyl; $C_1$-$C_{12}$-, bevorzugt $C_1$-$C_4$-Halogenalkyl, z.B. Fluor-oder Chlormethyl, Difluor- oder Dichlormethyl und Trifluor- oder Trichlormethyl; $C_1$-$C_4$-Alkoxy, z.B. Methoxy und Ethoxy; Halogen, z.B. Br, Cl und F; Phenyl; Phenoxy; oder wie zuvor für Alkyl erwähnt mit Sekundäramino. Bevorzugt sind $R^1$, $R^2$, $R^3$ und $R^4$ unsubstituiert.

In einer bevorzugten Untergruppe stehen $R^1$, $R^2$, $R^3$ und $R^4$ unabhängig voneinander für H oder unsubstituiertes oder substituiertes $C_1$-$C_6$-Alkyl oder Phenyl.

In Formel I stehen bevorzugt m und n für 1, oder m für 0 und n für 1, oder m für 1 und n für 0.

In einer bevorzugten Untergruppe stellt A zusammen mit der $-(CR^1R^2)_m NF-SO_2-(CR^3R^4)_n$ Gruppe einen 6- und besonders einen 5-gliedrigen Ring dar, wobei insbesondere m für 0 und n für 1 oder n für 0 und m für 1 stehen. In einer besonders bevorzugten Untergruppe stellt A einen wie zuvor definierten cycloaliphatischen Rest dar, an den die Gruppe $-NF-SO_2-CR^3R^4-$ in 1,2-Stellung gebunden ist, oder einen Arylen- oder Heteroarylenrest, an den die Gruppe $-CR^1R^2-NF-SO_2-$ in 1,2-Stellung gebunden ist.

Der Rest A kann ausgewählt sein aus der folgenden Gruppe; wobei die cyclischen Reste vorzugsweise in $\alpha,\beta$-Stellung an die Gruppe $-(CR^1R^2)_m NF-SO_2-(CR^3R^4)_n$ gebunden sind:

1. $C_1$-$C_6$-, besonders $C_1$-$C_4$- und insbesondere $C_1$- oder $C_2$-Alkylen, z.B. 1,6-Hexylen, 1,5-Pentylen, 1,4-Butylen, 1,3-Propylen und besonders Ethylen oder Methylen;

2. $C_2$-$C_6$-Alkenylen, besonders $C_2$-$C_4$-Alkylen, z.B. Hex-1-en-1,6-ylen, Hex-3-en-1,6-ylen, Pent-2-en-1,5-ylen, But-1-en- oder -2-en- oder -3-en- oder -4-en-1,4-ylen, Prop-1-en- oder -2-en- oder -3-en-1,3-ylen und besonders Ethenylen;

3. $C_4$-$C_6$-Alkdienylen, z.B. Buta-1,3-dien-1,4-ylen, Penta-1,3- oder -1,4-dien-1,5-ylen, Penta-2,4-dien-1,5-ylen, Hexa-1,3- oder -1,4- oder -1,5-dien-1,6-ylen, Hexa-2,4- oder -2,5-dien-1,6-ylen, Hexa-3,5-dien-1,6-ylen;

4. $C_6$-Alktrienylen, z.B. Hexa-1,3,5-trien-1,6-ylen;

5. $C_5$-$C_8$-Cycloalkylen, z.B. Cyclopentylen, Cylcohexylen, Cycloheptylen und Cyclooctylen, wobei Cyclopentylen und Cyclohexylen besonders bevorzugt sind;

6. $C_5$-$C_8$-, besonderes $C_5$- oder $C_6$-Cycloalkenylen, z.B. Cyclooctenylen, Cycloheptenylen, Cyclohex-1- oder -2- oder -3- oder -4-oder -5- oder -6-en-1,2-ylen, Cyclopent-1- oder -2- oder -3- oder -4- oder -5-en-1,2-ylen;

7. $C_5$-$C_8$-, besonders $C_5$- oder $C_6$-Cycloalkdienylen, z.B. Cyclooctadienylen, Cycloheptadienylen, Cyclohexa-1,3- oder -1,4- oder -1,5- oder -2,4- oder -2,5- oder -2,6- oder -3,5- oder -3,6-dien-1,2-ylen, Cyclopenta-1,3- oder -1,4- oder -2,4- oder -2,5- oder -3,5-dien-1,2-ylen;

8. $C_7$- oder $C_8$-Cycloalktrienylen, z.B. Cyclohepta-1,3,5- oder -2,4,6-oder -3,5,7-trien-1,2-ylen, Cycloocta-1,3,5- oder -2,4,6-oder -3,5,7-oder -4,6,8- oder -1,4,6- oder -1,5,7- oder -2,5,7-trien-1,2-ylen;

9. $C_6$-$C_{14}$-, besonders $C_6$-$C_{10}$-Arylen, besonders 1,2- oder 2,3-Naphthylen oder 1,2-Phenylen;

10. Heteroarylen mit 5 oder 6 Ringatomen und einem oder zwei, vorzugsweise einem der Heteroatome O, S und tertiärem N, wobei es sich bei dem tertiären N-Atom um die Gruppe =N- oder ein substituiertes, z.B. mit $C_1$-$C_4$-Alkyl, sekundäres N-Atom handeln kann. Beispiele für Heteroaryl, von denen sich der Rest A ableiten kann, sind Pyridin, Pyrimidin, Thiophen, Furan, Pyran, N-substituiertes Pyrrol oder Indol, Benzofuran, Benzothiophen, Chinolin und Isochinolin;

11. Die Reste Alkylen, Alkenylen Alkdienylen, Alktrienylen, Cycloalkylen, Cycloalkenylen, Cycloalkdienylen, Cycloalktrienylen können mit einem carbo- oder heterocyclischen Ring kondensiert sein, wobei der carbocyclische Ring vorzugsweise 5 oder 6 C-Atome enthält und es sich um einen cycloaliphatischen oder aromatischen Rest handeln kann, und wobei der heterocyclische Rest 2, bevorzugt 1 Heteroatom aus der Gruppe O, S und tertiäres N und 5 oder 6 Ringatome enthält und es sich um einen heterocycloaliphatischen oder heteroaromatischen Rest handeln kann. Bei dem tertiären N kann es sich um die Gruppe =N-oder um ein substituiertes, z.B. mit $C_1$-$C_4$-Alkyl, sekundäres N-Atom handeln. Beispiele für solche carbo- und heterocyclischen Ringe sind Cyclopentan, Cyclohexan, Cyclopenten, Cyclohexen, Cyclopentadien, Cyclohexadien, Benzol, Pyridin, Thiophen, Furan, N-substituiertes Pyrrol, Di- oder Tetrahydrofuran oder -thiophen, N-substituiertes Piperidin, Morpholin, Pyrrolidin oder Pyrrolin;

12. A in der Bedeutung eines cycloaliphatischen Restes kann mit einer Gruppe $-(CR^5R^6)_x$ überbrückt sein, worin $R^5$ und $R^6$ unabhängig voneinander bevorzugt für H, $CH_3$ oder $C_2H_5$ stehen und die Brückengruppe insbesondere Methylen, Ethyliden, 1,1- oder 2,2-Propyliden, Ethylen oder Methylethylen darstellt. Beispiele für solche überbrückten cycloaliphatischen Ringe mit vorzugsweise 6-14, besonders 6 bis 10 C-Atomen, von denen sich der Rest A ableiten kann, sind Bicyclo-[2,2,0]-hexan, Bicyclo-[2,2,1]-heptan, Bicyclo-[2,2,2]-octan, Bicyclo-[3,1,1]-heptan, Bicyclo-[3,2,1]-octan, Bicyclo-[3,3,0]-octan, Bicyclo-[3,4,0]-nonan, Bicyclo-[3,3,1]-nonan, Bicyclo-[2,2,1]-6,7,7-trimethylheptan, Bicyclo-[2,2,2]-7-methyloctan.

Der Rest A kann unsubstituiert oder ein- oder mehrfach, vorzugsweise ein- bis dreifach substituiert sein.

Geeignete Substituenten sind z.B. $C_1$-$C_6$-Alkyl, beispielsweise Methyl, Ethyl, n- oder i-Propyl, n-, i-oder t-Butyl, Pentyl, Hexyl; $C_1$-$C_6$-Alkoxy, besonders Methoxy oder Ethoxy; Sekundäramino, beispielsweise ($C_1$-$C_6$-Alkyl)$_2$N-, worin das Alkyl bevorzugt Methyl, Ethyl, Propyl oder Butyl ist; Phenyl; Phenoxy; Cl, Br und der Rest A in der Bedeutung eines cyclischen Restes auch mit F.

Eine bevorzugte Gruppe von Verbindungen der Formel I sind solche, worin in Formel I m und n 1 bedeuten und A für unsubstituiertes oder substituiertes $C_1$- oder $C_2$-Alkylen steht, das mit einem carbocylischen Ring kondensiert sein kann.

Eine andere Gruppe bevorzugter Verbindungen sind solche, worin m für 0 und n für 1 oder m für 1 und n für 0 stehen, und A unsubstituiertes oder substituiertes $C_2$-$C_3$-Alkenylen, $C_5$- oder $C_6$-Cycloalkylen, -Cycloalkenylen oder -Cycloalkdienylen bedeutet, die mit einem carbocyclischen Ring kondensiert oder die cyclischen Reste mit einer $\{CR^5R^6\}_x$ Gruppe überbrückt sein können, worin $R^5$ und $R^6$ unabhängig voneinander $C_1$-$C_4$-Alkyl oder H und x 1 oder 2 darstellen. Der carbocyclische Ring ist in diesen Gruppen besonders ein Naphthalin- oder Benzolring.

Bevorzugt sind ferner solche Verbindungen, worin in Formel I n für 0 und m für 1 oder m für 0 und n für 1 steht und der Rest A unsubstituiertes oder substituiertes 1,2-Naphtylen, 1,2-Phenylen oder 2,3-Pyridylen bedeutet.

Es wurde überraschend gefunden, dass die erfindungsgemässen Verbindungen stereoselektiv sind und in hohen Ausbeuten bei der Fluorierung von olefinischen Carbanionen nur das E- oder Z-Isomere bilden. Wenn überdies die erfindungsgemässen Verbindungen chiral sind, und in solchen Fällen in Form eines einzelnen optisch aktiven Stereoisomeren eingesetzt werden, dann wird bei der Fluorierung von sekundären und tertiären, aliphatischen Carbanionen überraschend in zum Teil sehr hohen optischen Ausbeuten nur ein Stereoisomeres gebildet.

Eine bevorzugte Untergruppe sind daher solche Verbindungen der Formel I, worin die C-Atome der $\{CR^1R^2\}$ Gruppe und/oder der $\{CR^3R^4\}$ Gruppe chiral sind, und/oder der Rest A mindestens ein chirales C-Atom enthält, in Form ihrer Racemate und/oder einzelnen Stereoisomeren.

Es wurde ferner gefunden, dass die optische Induktion dann besonders hoch ist, wenn sich das chirale C-Atom nahe bei der NF-Gruppe in Formel I befindet. Bevorzugter sind daher solche Verbindungen, worin in Formel I die chiralen C-Atome in $\alpha$- oder $\beta$-Position zur NF-Gruppe gebunden sind.

Eine andere Gruppe bevorzugter Verbindungen sind solche, worin in Formel I m 1 und n 0 ist, $R^1$ und $R^2$ die zuvor in Anspruch 1 angegebenen Bedeutungen haben und A für unsubstituiertes oder substituiertes 1,2-Phenylen steht, insbesondere solche chirale Verbindungen, worin $R^1$ und $R^2$ voneinander verschiedene Reste darstellen, in Form ihrer Racemate und Stereoisomeren. Eine besonders bevorzugte Verbindung ist 3-Deoxy-3,3-dimethyl-N-fluor-saccharin.

Ferner sind solche chirale Verbindungen bevorzugt, worin in Formel I m 0 und n 1 ist, $R^3$ und $R^4$ je für H stehen und A unsubstituiertes oder substituiertes, mindestens ein chirales C-Atom enthaltendes $C_5$-$C_6$-Cycloalkylen, das vorzugsweise mit Methylen, Ethyliden, 1,1-oder 2,2-Propyliden oder Ethylen überbrückt sein kann, in Form der Racemate oder der Stereoisomeren. Besonders bevorzugt sind unter diesen Verbindungen solche, worin A Cyclohexylen ist, das unsubstituiert oder in $\alpha$-Stellung zur NF-Gruppe mit $C_1$-$C_4$-Alkyl oder Phenyl substituiert ist und das mit Methylen, Ethyliden, 1,1- oder 2,2-Propyliden oder Ethylen überbrückt sein kann.

Insbesondere bevorzugt sind N-Fluorcamphersultame der Formel III

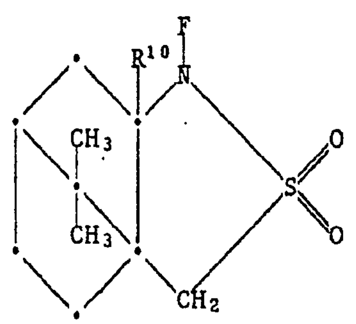

(III),

worin $R^{10}$ H, lineares oder verzweigtes $C_1$-$C_6$-Alkyl oder Phenyl darstellt, in Form der Racemate oder Stereoisomeren.

Bei einer besonders bevorzugten Verbindung handelt es sich um N-Fluorcamphersultam der Formel

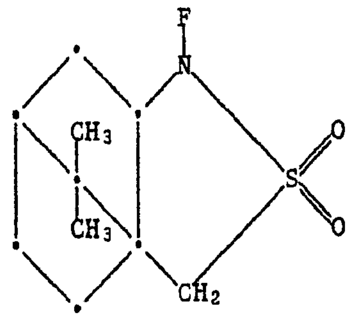

in Form seines Racemats oder der einzelnen Stereoisomeren.

Bei den Verbindungen der Formel I handelt es sich vorwiegend um kristalline Verbindungen, die im allgemeinen stabil und leicht zu handhaben sind. Bei den Verbindungen der Formel I, die in α-Position zur NF-Gruppe ein H-Atom enthalten, kann sich im Laufe der Zeit eine HF-Elimination bemerkbar machen, so dass sich ihre baldige Verwendung nach der Herstellung empfiehlt.

Die Verbindungen der Formel I können nach an sich bekannten Verfahren durch direkte Fluorierung von Verbindungen der Formel IV

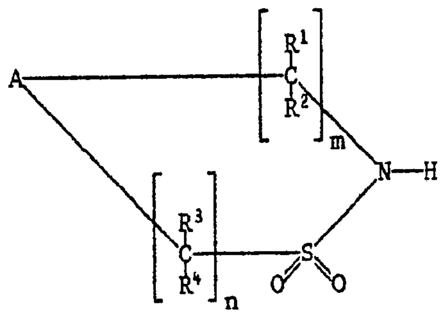

(IV),

worin $R^1$ bis $R^4$, m n und A die zuvor angegebene Bedeutung haben, erhalten werden. Ein entsprechendes Verfahren ohne Verwendung eines HF-Fängers ist in der US-PS 4 479 901 beschrieben.

Besonders geeignet ist die Direktfluorierung in Gegenwart eines HF-Fängers durchzuführen. Geeignete HF-Fänger sind z.B. Molekularsiebe oder Alkalimetallfluorid wie z.B. LiF, NaF, KF oder CsF, oder tertiäre Amine.

Eine Elektrofluorierung ist z.B. in der EP-A-0 211 578 beschrieben.

Die Verbindungen der Formel IV sind teilweise bekannt und käuflich, oder nach bekannten Verfahren herstellbar. Ein Verfahren ist z.B. in J. Chem. Soc., S. 1339 (1952) oder ein anderes in Helv. Chim. Acta, Vol. 67, S. 1397-1401 (1984) beschrieben. Camphersultam der Formel III ist als (-)-Enantiomer käuflich. Mit Alkylierungs- bzw. Phenylierungsreagenzien kann die Gruppe $R^{10}$ in der Bedeutung von $C_1$-$C_6$-Alkyl und Phenyl eingeführt werden.

Die bekannten Fluorierungsverfahren von N-H-Sulfonamiden haben den Nachteil, dass Nebenreaktionen die Ausbeute mindern. Es wurde überraschend gefunden, dass die direkte Fluorierung mit praktisch quantitativer Ausbeute innerhalb kurzer Reaktionszeiten möglich ist, wenn man in Gegenwart von HF-Fängern oder wenn man N-silylierte Sultame als Ausgangsprodukte verwendet.

Ein weiterer Gegenstand der Erfindung ist ein Verfahren zur Herstellung von Verbindungen der Formel I, das dadurch gekennzeichnet ist, dass man eine Verbindung der Formel II

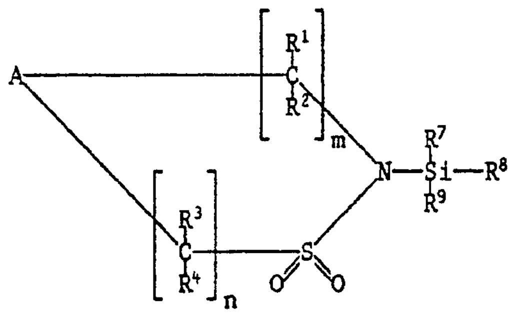

(II),

worin A, $R^1$, $R^2$, $R^3$, $R^4$, m und n die zuvor angegebene Bedeutung haben und $R^7$, $R^8$ und $R^9$ unabhängig voneinander lineares oder verzweigtes $C_1$-$C_{12}$-Alkyl, Cyclopentyl, Cyclohexyl, Benzyl oder Phenyl bedeuten, mit einem Fluorierungsmittel umsetzt.

Geeignete Fluorierungsmittel sind z.B. $F_2$, $CF_3OF$ oder $CH_3COOF$.

Die Verbindungen der Formel II sind ebenfalls ein Gegenstand der Erfindung. Sie sind in einfacher Weise nach bekannten Verfahren durch Silylierung von Verbindungen der Formel IV erhältlich. Solche Verfahren sind z.B. in E. Colvin, Silicon in Organic Synthesis, Butterworth, London (1981) oder Dissertation Hans Baumann, Nr. 7932, ETH Zürich (1985) beschrieben. Für $R^1$ bis $R^4$, A, m und n gelten die gleichen Bevorzugungen, wie sie zuvor für die Verbindungen der Formel I beschrieben sind. Bei $R^7$, $R^8$ und $R^9$ kann es sich unabhängig voneinander um lineares oder verzweigtes Alkyl mit vorzugsweise 1 bis 6, besonders 1 bis 4 C-Atomen handeln; besonders bevorzugt sind $R^7$, $R^8$ und $R^9$ Methyl.

Das Verfahren kann in Gegenwart eines Lösungsmittels durchgeführt werden. Geeignete Lösungsmittel sind z.B. chlorierte und/oder fluorierte Kohlenwasserstoffe oder Nitrile, z.B. Methylenchlorid, Chloroform, Tetrachlorkohlenstoff, 1,2-Dichlorethan,1,1,1-Trichlorethan, 1,1,2,2-Tetrachlorethan, Fluortrichlormethan, 1,1,1-Trifluor-2,2,2-trichlorethan, Hexafluorbenzol und Acetonitril.

Die direkte Fluorierung wird vorteilhaft mit einem Inertgas-Fluor-Gemisch durchgeführt. Geeignete Inertgase sind beispielsweise Edelgase wie z.B. Helium, Neon und Argon, oder Stickstoff. Der Fluoranteil im Gemisch kann 0,1 bis 99,9, vorzugsweise 0,5 bis 50 und besonders 1 bis 20 Vol.-% betragen.

Die Reaktionstemperatur kann -100 bis +80 °C, bevorzugt -80 bis +20 °C, und besonders -50 bis +20 °C betragen.

Es ist zweckmässig, die Reaktion unter Ausschluss von Sauerstoff und Feuchtigkeit durchzuführen.

Die Isolierung der gewünschten Verbindungen kann in üblicher Weise erfolgen, z.B. durch Verdampfen des Lösungsmittels und anschliessende Destillation, Sublimation oder Kristallisation. Das so erhaltene Produkt kann noch gereinigt werden, z.B. durch Umkristallisation oder mittels chromatographischer Methoden.

Die Verbindungen der Formel I sind hervorragende C-Fluorierungsmittel und somit wertvolle Reagenzien zur Herstellung von z.B. fluorierten biologisch aktiven Wirkstoffen. Besonders vorteilhaft ist die Möglichkeit zur stereoselektiven Fluorierung, da bekannterweise bestimmte Stereoisomere eine höhere biologische Aktivität aufweisen als ihre Antipoden.

Die C-Fluorierung ist z.B. in US-A-4 479 901, EP-A-O 211 578 und J. Am. Chem. Soc., Vol. 108, S. 2445-2447 beschrieben. Geeignete Kohlenstoffverbindungen sind z.B. Carbanionen. Geeignete Carbanionen können z.B. durch die allgemeine Formel $(C^{\ominus}{}_z M^{y\oplus} X_{y-z}$, worin C der organische Rest eines Carbanions, M ein Metall oder Halbmetall, X Halogen, besonders Cl, z eine Zahl von 1 bis 4 und y die Wertigkeit des Metalls ist, dargestellt werden. Beispiele für M sind Li, Na, K, Mg, Cd, Zn, Al, Ti, Zr, Si, M kann auch für quaternäres Ammonium stehen. Ferner können auch mit z.B. N, S, P oder Se stabilisierte Carbanionen (Ylide) mit den erfindungsgemässen Verbindungen fluoriert werden.

Die Fluorierung wird im allgemeinen in Gegenwart eines Lösungsmittels, vorteilhaft eines polaren aprotischen Lösungsmittels wie z.B. Ethern (Diethylether, Dibutylether, Tetrahydrofuran, Dioxan, Ethylenglykoldimethylether) und bei Temperaturen von z.B. -100 °C bis 100 °C, besonders -100 °C bis 50 °C, insbesondere -80 °C bis 20 °C durchgeführt.

Ein weiterer Gegenstand der Erfindung ist die Verwendung der Verbindungen der Formel I zur C-Fluorierung, insbesondere von Verbindungen der Formel I in Form eines optisch aktiven Stereoisomeren einer chrialen Verbindung zur stereoselektiven C-Fluorierung.

Die nachfolgenden Beispiele erläutern die Erfindung näher.

A) Herstellungsbeispiele

Beispiel 1: N-Trimethylsilyl-3-deoxy-3,3-dimethyl-saccharin

100 mMol 3-Deoxy-3,3-dimethylsaccharin [hergestellt nach der Vorschrift in J. Chem. Soc., S. 1339 (1952)] werden nach der Vorschrift von Hans Baumann, Dissertation Nr. 7932, ETH Zürich (1985) unter Argonatmosphäre in einem Ueberschuss Hexamethyldisilazan während 20 Stunden am Rückfluss erhitzt. Danach wird am Rotationsverdampfer bei 70 °C unter reduziertem Druck eingedampft und der Rückstand über Nacht im Hoch vakuum getrocknet. Man erhält in praktisch quantitativer Ausbeute N-Trimethylsilyl-3-deoxy-3,3-dimethylsaccharin als weisses Pulver mit einem Schmelzpunkt (Smp.) von 144-146 °C.

Beispiel 2: (-)-N-Trimethylsilylcamphersultam

Es wird wie im Beispiel 1 verfahren, aber (-)-Camphersultam (käuflich, Firma Aldrich) an Stelle des Saccharins verwendet. Man erhält in praktisch quantitativer Ausbeute (-)-N-Trimethylsilylcamphersultam als hellbeige Kristalle vom Smp. 63-73 °C.

Beispiel 3: N-Fluor-3-deoxy-3,3-dimethylsaccharin

Unter Luft- und Feuchtigkeitsausschluss werden 40 mMol der Verbindung gemäss Beispiel 1 in einem Gemisch aus 250 ml $CHCl_3/CFCl_3$ (3:2) während 3 Stunden bei -40 °C mit einem Gemisch aus 10 Vol.-% Fluor in Stickstoff umgesetzt. Anschliessend wird bei Raumtemperatur mit $N_2$ gespült, am Rotationsverdampfer unter vermindertem Druck eingeengt und der Rückstand aus Ether/Pentan umkristallisiert. Man erhält in praktisch quantitativer Ausbeute N-Fluor-3-deoxy-3,3-dimethylsaccharin als weisse Kristallnadeln mit einem Smp. von 114-116 °C.

Beispiel 4: (-)-N-Fluorcamphersultam

Unter Verwendung der Verbindungen gemäss Beispiel 2 wird wie in Beispiel 3 verfahren, aber zusätzlich vor der Umkristallisation in $CH_2Cl_2$ über Kieselgel chromatographiert. Auf den Umsatz bezogen erhält man in praktisch quantitativer Ausbeute (-)-N-Fluor-camphersultam als weisse Kristalle mit einem Smp. von 104 °C und einer Drehung $[\alpha]_D^{20}$ von -10,64 ° (C = 0,7, $CHCl_3$).

Beispiel 5: N-Fluor-3-deoxy-3,3-dimethylsaccharin

Unter Luft- und Feuchtigkeitsausschluss werden 25 mMol 3-Deoxy-3,3-dimethylsaccharin in einem Gemisch aus 200 ml $CHCl_3/CFCl_3$ (1:1) während 20 min bei -40 °C mit einem Gemisch aus 10 Vol.-% Fluor in Stickstoff in Gegenwart von NaF umgesetzt. Anschliessend wird wie in Beispiel 3 aufgearbeitet; Ausbeute: 4,0 g (74 %) N-Fluor-3-deoxy-3,3-dimethylsaccharin vom Smp. 114-116 °C.

Beispiel 6: (±)-N-Fluor-3-t-butyl-3-deoxy-3-methylsaccharin

a) 1,2-Benzisothiazol-3-t-butyl-1,1-dioxid

Die Umsetzung von 1,2-Benzisothiazol-3-chlor-1,1-dioxid mit t-Butyl-magnesiumchlorid in absolutem Tetrahydrofuran (THF) bei Raumtemperatur ergibt 1,2-Benzisothiazol-3-t-butyl-1,1-dioxid als leicht gelbliche Kristalle vom Smp. 105-110 °C (Umkristallisation aus Diethylether).

b) (±)-3-t-Butyl-3-deoxy-3-methylsaccharin

Die Umsetzung von Verbindung gemäss a) mit Methyllithium in absolutem THF bei -78 °C ergibt (±)-3-t-Butyl-3-deoxy-3-methylsaccharin als farblose Kristalle vom Smp. 170-172 °C (Umkristallisation aus Toluol).

c) (±)-N-Fluor-3-t-butyl-3-deoxy-3-methylsaccharin

Unter Verwendung der Verbindung gemäss b) wird wie in Beispiel 5 verfahren. Aufgearbeitet wird wie in Beispiel 3, wobei das ölige Rohprodukt mit Hexan/Essigester (5:1) über Kieselgel chromatographiert wird. Ausbeute: 83 % farbloses Oel.
$^1$H-NMR (300 MHz, $CDCl_3$): 1,14 ppm (d,9H), 1,65 ppm (d, 3H), 7,55-7,80 (m, 4H).

Beispiel 7: (±)-N-Fluor-2-methylcamphersultam

a) (-)-2-Methylcamphersultam

Das von Oppolzer et al. in Tetrahedron 42, 4035-4043 (1986) beschriebene (-)-Camphersulfonimid wird mit Methylmagnesiumjodid in Gegenwart von 1 äquiv. Kupfer(I)chlorid in Diethylether bei Raumtemperatur methyliert und ergibt einheitliches (-)-2-Methyl-oamphpersultam als farblose Kristalle vom Smp. 240-243 °C und einer Drehung $[\alpha]_D^{20}$ von -36,1 ° (C = 1,00, $CHCl_3$).

b) (+)-N-Fluor-2-methylcamphersultam

Unter Verwendung der Verbindung gemäss a) wird wie in Beispiel 5 verfahren. Nach Filtration und Einengen am Rotationsverdampfer wird aus $CH_2Cl_2$ umkristallisiert. Ausbeute: 80 % farblose Kristalle vom Smp. 151-154 °C und einer Drehung $[\alpha]_D^{20}$ von +10,27 (C = 0,74, $CHCl_3$).

B) Anwendungsbeispiele

Beispiel 8: Herstellung von E-1-Fluor-1-octen.

Eine Lösung von 4 mmol des E-1-Jod-1-octen in 30 ml Tetrahydrofuran wird bei -78 °C innert 30 Minuten mit 5,7 ml (8 mmol) tert.-Butyllithium (1,4 M Lösung in Pentan) und nach weiteren 30 Minuten mit der Lösung von 6 mmol N-Fluor-3-deoxy-3,3-dimethylsaccharin (Beispiel 3) in 6 ml Tetrahydrofuran versetzt. Es wird über Nacht gerührt und danach flüchtige Produkte zusammen mit dem Lösungsmittel im Hochvakuum abgezogen und in einer mit flüssigem Stickstoff gekühlten Vorlage kondensiert. Das Kondensat wird fraktioniert destilliert. Man erhält ein Gemisch aus 1-Octen und E-1-Fluor-1-octen, das bei 80 °C siedet.

Beispiel 9: Herstellung von E- und Z-1-Fluor-4-thexyldimethylsilyloxy-1-buten.

a) Thexyldimethylsilyloxy-1-butin

Eine Lösung von 0,37 Mol 1,8-Diazabicyclo-[5,4,0]-undec-7-en und 0,36 Mol Thexyldimethylchlorsilan in 300 ml Methylenchlorid wird innerhalb von 10 Minuten mit 0,338 Mol 3-Butin-1-ol versetzt. Das Gemisch wird nach 15 Minuten mit Wasser. 0,1 N HCl und Natriumbicarbonatlösung gewaschen, über $Na_2SO_4$ getrocknet, filtriert und eingedampft. Die Destillation des Rückstands ergibt das gewünschte Produkt vom Siedepunkt 86-89 °C/20 mbar.

b) 4-Thexyldimethylsilyloxy-1-tributylstannyl-1-buten (E/Z = 4:1)

Eine Mischung von 10,6 g (0,05 Mol) des Alkins (a) und 14,6 ml (0,055 mol) Tributylzinnhydrid wird über nacht bei -10° bis 0°C mit einer 200 W Glühlampe bestrahlt und danach das Produkt durch Destillation isoliert. Man erhält 23 g Verbindung b) vom Siedepunkt 126-145°C/0,01 mbar.

c) E- und Z-1-Fluor-4-thexyldimethylsilyloxy-1-buten

Eine Lösung von 3mMol (b) in 22 ml eines Lösungsmittelgemisches aus THF/Diethylether/Pentan (4:1:1) wird bei -70°C mit 2,25 ml (3,6 mmol) Butyl-Lithium (1,6 M in Hexan) versetzt und 45 Minuten bei dieser Temperatur belassen. Danach wird auf -120°C abgekühlt und mit einer Lösung von 3 mmol N-Fluor-3-deoxy-3,3-dimethylsaccharin (Beispiel 3) in 3 ml THF versetzt. Nach einer Stunde bei -120° bis -110°C wird langsam auf Raumtemperatur erwärmt, mit Wasser versetzt und mit Hexan extrahiert. Nach Trocknen, Eindampfen und Flash-chromatographie an Kieselgel mit Cyclohexan erhält man die Titelverbindung als E/Z-Gemisch (4:1).
$^1$H-NMR (300 MHz. $CDCl_3$); E-Isomer: 6,52 (C-1H); 5,35 (C-2H); 2,33 (C-3H); 3,62 (C-4H). Z-Isomer: 6,47 (C-1H); 4,80 (C-2H); 2,09 (C-3H); 3,62 (C-4H).

Beispiel 10: Herstellung von (+)-1-Fluor-1-carbethoxycyclopentan-2-on.

Zu einer Suspension von 1,56 mmol Natriumhydrid in 5 ml Tetrahydrofuran gibt man bei 0°C 1,3 mmol 1-Carbethoxycyclopentan-2-on und rührt 1 Stunde bei 0°C. Anschliessend tropft man eine Lösung von 1,4 mmol (-)-N-Fluorcamphersultam (Beispiel 4) zu und rührt 3 Stunden bei 0°C weiter. Dann giesst man das Reaktionsgemisch in 50 ml 1 M Oxalsäure, extrahiert mit Diethylether und wäscht die organischen Phasen mit 10 %-iger $NaHCO_3$-Lösung und gesättigter NaCl-Lösung. Das erhaltene Gemisch wird auf einer Kieselgelsäule gereinigt. Man erhält (+)-1-Fluor-1-carbethoxycyclopentan-2-on in guter Enantiomereneinheit (ee = 70 %).
$^1$H-NMR: (300 MHz, $CDCl_3$): 1,32 ppm (t, 3 H,), 2,16 ppm (m, 2 H); 2,31 ppm (m, 1 H); 2,50 ppm (t, 2 H); 2,55 ppm (m, 1 H); 4,30 ppm (q, 2 H).

Beispiel 11: Herstellung von (+)-1-Fluor-1-carbethoxycyclopentan-2-on.

Gemäss Beispiel 10 jedoch mit Kaliumhydrid in Toluol/THF (2:1) wird das Enolat von 1 Carbethoxycyclopentan-2-on gebildet und mit (+)-N-Fluor-2-methylcamphersultam (Beispiel 7) fluoriert. Die Enantiomerenreinheit im gewünschten Produkt: ee = ca. 5 %.

Beispiel 12: Herstellung von 2-Fluor-2-methylacetessigsäure-ethylester.

Gemäss Beispiel 10 jedoch mit Lithiumhydrid in Diethylether wird das Enolat von 2-Methylacetessigsäure-ethylester gebildet und bei Raumtemperatur mit (-)-N-Fluorcamphersultam (Beispiel 4) fluoriert. Die Enantiomerenreinheit im gewünschten Produkt: ee = ca. 10 %.

Beispiel 13: Herstellung von 2-Fluor-2-phenylpropionsäure-ethylester.

Gemäss Beispiel 10 jedoch mit Lithiumdimethylamid in THF bei 78°C wird das Enolat von 2-Phenylpropionsäure-ethylester gebildet und bei Raumtemperatur mit (-)-N-Fluorcamphersultam (Beispiel 4) fluoriert. Die Enantiomerenreinheit im gewünschten Produkt: ee = ca. 35 %.

Beispiel 14: Herstellung von 2-Fluor-2-phenylpropionsäure-ethylester.

Gemäss Beispiel 13 wird das entsprechende Enolat gebildet und bei Raumtemperatur mit (+)-N-Fluor-2-methylcamphersultam (Beispiel 7) fluoriert. Die Enantiomerenreinheit im gewünschten Produkt: ee = ca. 10 %.

Beispiel 15: Herstellung von 2-Fluor-2-methyl-1-oxo-1,2,3,4-tetrahydronaphtalin.

Gemäss Beispiel 13 wird das Enolat aus 2-Methyl-1-oxo-1,2,3,4-tetrahydronaphtalin gebildet und bei Raumtemperatur mit (-)-N-Fluorcamphersultam (Beispiel 4) fluoriert. Die Enantiomerenreinheit im gewünschten Produkt: ee = ca. 35 %.

Beispiel 16: Herstellung von (±)-2-Fluor-2-phenylpropionsäure-methylester.

Gemäss Beispiel 14 wird das entsprechende Enolat gebildet und bei Raumtemperatur mit N-Fluor-3-deoxy-3,3-dimethylsaccharin fluoriert. Ausbeute: 70 %.
$^1$H-NMR (300 MHz, $CDCl_3$): 1,91 (d, 3H), 3,74 (s, 3H), 7,30-7,53 (m, 5H).

Beispiel 17: Herstellung von (±)-2-Fluor-2-phenylpropionsäure-methylester.

Gemäss Beispiel 14 wird das entsprechende Enolat gebildet und bei Raumtemperatur mit (±)-N-Fluor-3-t-butyl-3-deoxy-3-methylsaccharin (Beispiel 6) fluoriert. Ausbeute: 43 %.

## Ansprüche

1. Verbindungen der Formel I

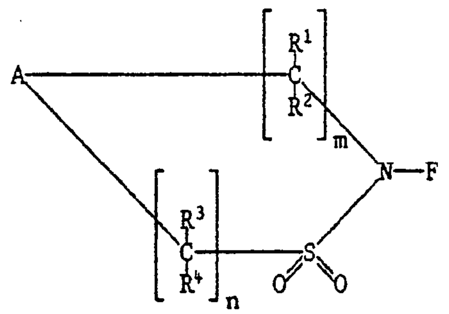

(I),

worin
$R^1$, $R^2$, $R^3$ und $R^4$ unabhängig voneinander für H, lineares oder verzweigtes $C_1$-$C_{18}$-Alkyl oder $C_6$-$C_{10}$-Aryl stehen, die unsubstituiert sind oder das Alkyl mit Halogen, $C_6$-$C_{10}$-Aryl oder -Aryloxy, $C_1$-$C_6$-Alkoxy oder Sekundäramino und das Aryl mit $C_1$-$C_{12}$-Alkyl, -Halogenalkyl oder -Alkoxy, Halogen, Phenyl, Phenoxy oder Sekundäramino substituiert ist, m und n unabhängig voneinander für 0 oder 1 stehen, und A einen zweiwertigen organischen Rest bedeutet, der mit der $-(CR^1R^2)_m NF-SO_2-(CR^3R^4)_n$ Gruppe einen 5- bis 8-gliedrigen Ring bildet und der Rest A ausgewählt ist aus der Gruppe

a) $C_1$-$C_6$-Alkylen, $C_2$-$C_6$-Alkenylen, $C_4$-$C_6$-Alkdienylen, $C_6$-Alktrienylen, $C_5$-$C_8$-Cycloalkylen, $C_5$-$C_8$-Cycloalkenylen, $C_5$-$C_8$-Cycloalkdienylen oder $C_7$-$C_8$-Cycloalktrienylen, die mit einem carbo- oder heterocyclischen Ring kondensiert oder die cycloaliphatischen Reste mit einer $-(CR^5R^6)_x$ Gruppe überbrückt sein können, worin $R^5$ und $R^6$ unabhängig H oder $C_1$-$C_4$-Alkyl darstellen und x für 1 oder 2 steht,

b) $C_6$-$C_{14}$-Arylen oder Heteroarylen mit 5 oder 6 Ringatomen und 1 oder 2 der Heteroatome O, S und tertiäres N, wobei der Rest A unsubstituiert oder ein- oder mehrfach mit $C_1$-$C_6$-Alkyl oder -Alkoxy, Sekundäramino, Phenyl, Phenoxy, Cl, Br und der Rest A als cyclischer Rest auch mit F substituiert ist.

2. Verbindungen gemäss Anspruch 1, worin in Formel I m und n für 1, oder m für 0 und n für 1 oder n für 0 und m für 1 stehen.

3. Verbindungen gemäss Anspruch 1, worin in Formel I A zusammen mit der $-(CR^1R^2)_m NF-SO_2-(CR^3R^4)_n$ Gruppe einen 6- und besonders einen 5-gliedrigen Ring bildet.

4. Verbindungen gemäss Anspruch 3, worin m für 0 und n für 1 oder n für 0 und m für 1 stehen.

5. Verbindungen gemäss Anspruch 1, worin in Formel I m und n 1 bedeuten und A für unsubstituiertes oder substituiertes $C_1$- oder $C_2$-Alkylen steht, das mit einem carbocyclischen Ring kondensiert sein kann.

6. Verbindungen gemäss Anspruch 1, worin m für 0 und n für 1 oder m für 1 und n für 0 stehen, und A unsubstituiertes oder substituiertes $C_2$-$C_3$-Alkenylen, $C_5$- oder $C_6$-Cycloalkylen, -Cycloalkenylem oder -Cycloalkdienylen bedeutet, die mit einem carbocyclischen Ring kondensiert oder die cyclischen Reste mit einer $\{CR^5R^6\}_x$ Gruppe überbrückt sein können, worin $R^5$ und $R^6$ unabhängig voneinander $C_1$-$C_4$-Alkyl oder H und x 1 oder 2 darstellen.

7. Verbindungen gemäss den Ansprüchen 5 oder 6, worin es sich bei dem carbocyclischen Ring um einen Naphthalin- oder Benzolring handelt.

8. Verbindungen gemäss Anspruch 6, worin es sich bei der $\{CR^5R^6\}_x$ Gruppe um Methylen, Ethyliden, 1,1- oder 2,2-Propyliden, Ethylen oder Methylethylen handelt.

9. Verbindungen gemäss Anspruch 1, worin in Formel I n für 0 und m für 1 oder m für 0 und n für 1 steht und der Rest A unsubstituiertes oder substituiertes 1,2-Naphtylen, 1,2-Phenylen oder 2,3-Pyridylen bedeutet.

10. Verbindungen gemäss Anspruch 1, worin in Formel I $R^1$, $R^2$, $R^3$ und $R^4$ unabhängig voneinander für H stehen oder unsubstituiertes oder substituiertes $C_1$-$C_6$-Alkyl oder Phenyl bedeuten.

11. Verbindungen gemäss Anspruch 1 in Form ihrer Racemate oder einzelnen Stereoisomeren, worin in Formel I die C-Atome der $\{CR^1R^2\}$ Gruppe und/oder der $\{CR^3R^4\}$ Gruppe chiral sind, und/oder der Rest A mindestens ein chirales C-Atom enthält.

12. Verbindungen gemäss Anspruch 11, worin in Formel I die chiralen C-Atome in $\alpha$- oder $\beta$-Position zur NF-Gruppe gebunden sind.

13. Verbindungen gemäss Anspruch 9, worin in Formel I m 1 und n 0 ist, $R^1$ und $R^2$ die zuvor in Anspruch 1 angegebenen Bedeutungen haben und A für unsubstituiertes oder substituiertes 1,2-Phenylen steht.

14. Verbindungen gemäss Anspruch 13, worin $R^1$ und $R^2$ verschiedene Reste darstellen in Form ihrer Racemate oder Stereoisomeren.

15. Verbindungen gemäss Anspruch 13, worin es sich um 3-Deoxy-3,3-dimethyl-N-fluor-saccharin handelt.

16. Verbindungen gemäss Anspruch 1, worin in Formel I m 0 und n 1 ist, $R^3$ und $R^4$ je für H stehen und A unsubstituiertes oder substituiertes, mindestens ein chirales C-Atom enthaltendes $C_5$-$C_6$-Cycloalkylen, das vorzugsweise mit Methylen, Ethyliden, 1,1-oder 2,2-Propyliden oder Ethylen überbrückt sein kann, in Form der Racemate oder der einzelnen Stereoisomeren.

17. Verbindungen gemäss Anspruch 16, worin A Cyclohexylen ist, das unsubstituiert oder in $\alpha$-Stellung zur NF-Gruppe mit $C_1$-$C_4$-Alkyl oder Phenyl substituiert ist und das mit Methylen, Ethyliden, 1,1-oder 2,2-Propyliden oder Ethylen überbrückt sein kann.

18. Verbindungen gemäss Anspruch 16, worin es sich um N-Fluorcamphersultam der Formel

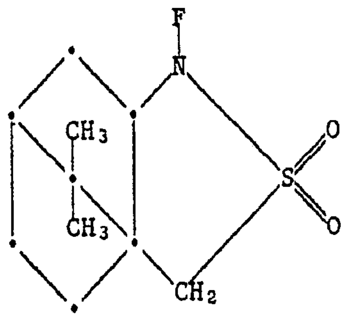

handelt in Form seines Racemats oder der einzelnen Stereoisomeren.

19. Verfahren zur Herstellung von Verbindungen der Formel I gemäss Anspruch 1, dadurch gekennzeichnet, dass man eine Verbindung der Formel II

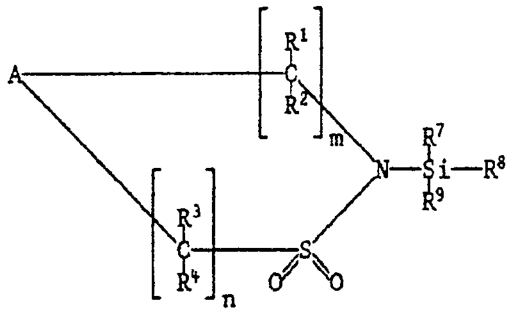

(II),

worin A, $R^1$, $R^2$, $R^3$, $R^4$, m und n die in Anspruch 1 angegebene Bedeutung haben und $R^7$, $R^8$ und $R^9$ unabhängig voneinander lineares oder verzweigtes $C_1$-$C_{12}$-Alkyl, Cyclopentyl, Cyclohexyl, Benzyl oder Phenyl bedeuten, mit einem Fluorierungsmittel umsetzt.

20. Verbindungen der Formel II

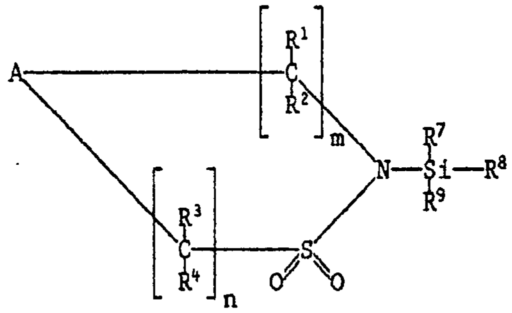

(II),

worin
$R^1$, $R^2$, $R^3$ und $R^4$ unabhängig voneinander für H, lineares oder verzweigtes $C_1$-$C_{18}$-Alkyl oder $C_6$-$C_{10}$-Aryl stehen, die unsubstituiert sind oder das Alkyl mit Halogen, $C_6$-$C_{10}$-Aryl oder -Aryloxy, $C_1$-$C_6$-Alkoxy oder Sekundäramino und das Aryl mit $C_1$-$C_{12}$-Alkyl, -Halogenalkyl oder -Alkoxy, Halogen, Phenyl, Phenoxy oder Sekundäramino substituiert ist. m und n unabhängig voneinander für 0 oder 1 stehen, und A einen zweiwertigen organischen Rest bedeutet, der mit der $\{CR^1R^2\}_mNF\text{-}SO_2\{CR^3R^4\}_n$ Gruppe einen 5- bis 8-gliedrigen Ring bildet und der Rest A, ausgewählt ist aus der Gruppe

a) $C_1$-$C_6$-Alkylen, $C_2$-$C_6$-Alkenylen, $C_4$-$C_6$-Alkdienylen, $C_6$-Alktrienylen, $C_5$-$C_8$-Cycloalkylen, $C_5$-$C_8$-Cycloalkenylen, $C_5$-$C_8$-Cycloalkdienylen oder $C_7$-$C_8$-Cycloalktrienylen, die mit einem carbo- oder heterocyclischen Ring kondensiert oder die cycloaliphatischen Reste mit einer $\{CR^5R^6\}_x$ Gruppe überbrückt sein können, worin $R^5$ und $R^6$ unabhängig H oder $C_1$-$C_4$-Alkyl darstellen und x für 1 oder 2 steht,

b) $C_6$-$C_{14}$-Arylen oder Heteroarylen mit 5 oder 6 Ringatomen und 1 oder 2 der Heteroatome O, S und tertiäres N, wobei der Rest A unsubstituiert oder ein- oder mehrfach mit $C_1$-$C_5$-Alkyl oder -Alkoxy, Sekundäramino, Phenyl, Phenoxy, Cl, Br und der Rest A als cyclischer Rest auch mit F substituiert ist und $R^7$, $R^8$ und $R^9$ unabhängig voneinander lineares oder verzweigtes $C_1$-$C_{12}$-Alkyl, Cyclopentyl, Cyclohexyl, Benzyl oder Phenyl bedeuten.

21. Verwendung von Verbindungen der Formel I gemäss Anspruch 1 zur C-Fluorierung.

22. Verwendung gemäss Anspruch 25, wobei die Verbindung der Formel I ein optisch aktives Stereoisomer einer chiralen Verbindung ist, zur stereoselektiven C-Fluorierung.

Europäisches Patentamt

# EUROPÄISCHER RECHERCHENBERICHT

Nummer der Anmeldung

EP 88 11 6596

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | EP-A-0 211 578 (RESEARCH CORP.)<br>* Ansprüche *<br>--- | 1,21,22 | C 07 D 275/06<br>C 07 F 7/10<br>C 07 B 39/00<br>C 07 D 513/04<br>C 07 D 279/02<br>C 07 D 281/02 |
| D,A | US-A-4 479 901 (W.E. BARNETTE)<br>* Ansprüche *<br>--- | 1,21,22 | |
| A | JOURNAL OF ORGANIC CHEMISTRY, Band 47, Nr. 20, 24. September 1982, Seiten 3966-3969, American Chemical Society, Washington, D.C., US; C.A. BRUYNES et al.: "Catalysts for silylations with 1,1,1,3,3,3-hexamethyldisilazane"<br>* Seiten 3967-3968 *<br>----- | 20 | |
| | | | **RECHERCHIERTE SACHGEBIETE (Int. Cl.4)** |
| | | | C 07 D 275/00<br>C 07 D 513/00 |

Der vorliegende Recherchenbericht wurde für alle Patentansprüche erstellt

| Recherchenort | Abschlußdatum der Recherche | Prüfer |
|---|---|---|
| DEN HAAG | 06-12-1988 | HENRY J.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
.......................................................
& : Mitglied der gleichen Patentfamilie, übereinstimmendes Dokument

EPO FORM 1503 03.82 (P0403)